# EUROPEAN PATENT SPECIFICATION

(11) **EP 1 237 553 B1**
(45) Date of publication and mention of the grant of the patent: **04.01.2006**
(21) Application number: 00981174.6
(22) Date of filing: 06.12.2000
(51) Int. Cl.: A61K 31/517, A61K 31/519, A61K 31/55, A61K 31/135, A61K 31/137, A61K 31/15, A61K 31/343, A61K 31/4525

(54) **THE COMBINATION OF A SEROTONIN REUPTAKE INHIBITOR AND A 5-HT 2C? ANTAGONIST, INVERSE AGONIST OR PARTIAL AGONIST**
KOMBINATION AUS EINEM SEROTONIN-WIEDERAUFNAHME-INHIBITOR UND EINEM 5-HT2C-ANTAGONIST, -INVERSE AGONIST ODER EINEM -PARTIELLEN AGONISTEN
COMBINAISON D'UN INHIBITEUR DU RECAPTAGE DE LA SEROTONINE ET D'UN ANTAGONISTE, D'UN AGONISTE INVERSE OU D'UN AGONISTE PARTIEL DE 5-HT 2C?

(30) Priority: 06.12.1999 US 169245 P
(43) Date of publication of application: 11.09.2002
(60) Divisional application: 03027672.9
(73) Proprietor: H. LUNDBECK A/S, 2500 Kobenhavn-Valby (DK)
(72) Inventor: CREMERS, Thomas, Ivo, Franciscus, Hubert, NL-9717 KV Groningen (NL); WIKSTRÖM, Hakan, Wilhelm, NL-9721 WN Groningen (NL); DEN BOER, Johan, Antonie, NL-9752 CH Haren (NL); BOSKER, Fokko, Jan, NL-9711 KS Groningen (NL); WESTERINK, Bernard, Hendrik, Cornelis, NL-9774 PL Adorp (NL); BOGESO, Klaus, Peter, DK-2970 Horsholm (DK); HOGG, Sandra, DK-1871 Frederiksberg C (DK); MORK, Arne, DK-3060 Espergaerde (DK)
(74) Representative: Meidahl Petersen, John
(86) International application number: PCT/DK2000/000671
(87) International publication number: WO 2001/041701

(56) References cited:
- EP-A1- 0 813 873
- EP-A1- 0 966 976
- DE-A1- 19 900 673
- GB-A- 2 303 303
- US-A- 5 786 157
- US-A- 5 958 429
- L. LAURITZEN ET AL.: 'Combined treatment with imipramine and mianserin' PHARMACOPSYCHIAT. vol. 25, 1992, pages 182 - 186, XP002951440
- MICHAEL MAES ET AL.: 'Pindolol and mianserin augment the antidepressant activity of fluoxetine in hospitalized major depressed patients, including those with treatment resistance' JOURNAL OF CLINICAL PSYCHOPHARMACOLOGY vol. 19, no. 2, 1999, pages 177 - 182, XP001055722
- PAUL J. GOODNICK ET AL.: 'New antidepressant agents: Recent pharmacological developments leading to improved efficacy' EXP. OPIN. INVEST. DRUGS vol. 4, no. 10, 1995, pages 935 - 943, XP001018332
- CONNIE SANCHEZ ET AL.: 'Comparison of the effects of antidepressants and their metabolites on reuptake of biogenic amines and on receptor binding' CELLULAR AND MOLECULAR NEUROBIOLOGY vol. 19, no. 4, 1999, pages 467 - 489, XP000861810
- AGNES BESSON: 'Effects of the co-administration of mirtazapine and paroxetine on serotonergic neurotransmission in the rat brain' EUROPEAN NEUROPSYCHOPHARMACOLOGY vol. 10, 2000, pages 177 - 188, XP001055741
- LAAK ET AL: "The Histamine H1 receptor antagonists binding site" J.MED.CHEM, vol. 38, 1995, pages 33651-3360,

## Description

The present invention relates to the use of certain compounds, and to compositions of compounds having selective serotonin reuptake inhibiting activity and 5-HT_{2C} antagonistic, partial agonistic or inverse agonistic activity for the treatment of depression and other affective disorders. The combined serotonin reuptake inhibiting effect and the 5-HT_{2C} antagonistic, partial agonistic or inverse agonistic effect may reside within the same chemical entity or in two separate chemical entities.

### Background

Selective serotonin reuptake inhibitors (hereinafter referred to as SSRIs) have become first choice therapeutics in the treatment of depression, certain forms of anxiety and social phobias, because they are effective, well tolerated and have a favourable safety profile compared to the classic tricyclic antidepressants.

However, clinical studies on depression indicate that non-response to SSRIs is substantial, up to 30%. Another, often neglected, factor in antidepressant treatment is compliance, which has a rather profound effect on the patient's motivation to continue pharmacotherapy.

First of all, there is the delay in therapeutic effect of SSRIs. Sometimes symptoms even worsen during the first weeks of treatment. Secondly, sexual dysfunction is a side effect common to all SSRIs. Without addressing these problems, real progress in the pharmacotherapy of depression and anxiety disorders is not likely to happen.

In order to cope with non-response, psychiatrists sometimes make use of augmentation strategies. Augmentation of antidepressant therapy may be accomplished through the co-administration of mood stabilizers such as lithium carbonate or triiodothyronin or by the use of electroshock.

The effect of combined administration of a compound that inhibits serotonin reuptake and a 5-HT_{1A} receptor antagonist has been evaluated in several studies (Innis, R.B. et al. *Eur. J. Pharmacol.* **1987,** *143,* p. 1095-204 and Gartside, S.E. *Br. J. Pharmacol.* **1995,** *115,* p 1064-1070, Blier, P. et al. *Trends in Pharmacol. Science* **1994**, *15*, 220). In these studies it was found that 5-HT_{1A} receptor antagonists would abolish the initial brake on 5-HT neurotransmission induced by the serotonin reuptake inhibitors and thus produce an immediate boost of 5-HT transmission and a rapid onset of therapeutic action.

Several patent applications have been filed which cover the use of a combination of a 5-HT_{1A} antagonist and a serotonin reuptake inhibitor for the treatment of depression (see e.g. EP-A2-687 472 and EP-A2-714 663).

Another approach to increase terminal 5-HT would be through blockade of the 5-HT_{1B} autoreceptor. Microdialysis experiments in rats have indeed shown that increase of hippocampal 5-HT by citalopram is potentiated by GMC 2-29, an experimental 5-HT_{1B} receptor antagonist.

Several patent applications covering the combination of an SSRI and a 5-HT_{1B} antagonist or partial agonist have also been filed (WO 97/28141, WO 96/03400, EP-A-701819 and WO 99/13877).

It has been demonstrated that 5-HT_{2C} ligands can affect the release of dopamine (DA) and noradrenaline (NE) in rat frontal cortex. Thus, the 5-HT_{2C} agonist Ro 60-0175 and the selective 5-HT_{2C} antagonist SB-242084 suppressed and increased levels of both DA and NE, respectively, without modifying those of serotonin (5-HT) (Millan, M.J. et al. *Neuropharmacology* **1998,** 37, p 953-955). A similar observation was done with the selective 5-HT_{2B/2C} antagonist, SB-206553 (Gobert, A. et al. *Neuropharmacology* **1999,** 38, p 315-317). Activation of 5-HT_{2C} receptors with the selective agonist MK-212 inhibited morphine-induced DA release in rat nucleus accumbens (Willins, D.L. et al. *Brain Res.* **1998** 781, p 291-299). A previous report demonstrated that quipazine-induced decrease of NE release in rat hippocampus was antagonised by ritanserin (Done, C.J. et al. Br J Pharmacol. **1992,** *107,* p 240-245). Thus, 5-HT_{2C} receptors seem to exert an influence upon release of NE and DA, but not of 5-HT. In the studies of Milan et al. (1998, 1999), 5-HT_{2C} ligands were injected alone and no effect was found on 5-HT release.

It is known that citalopram and fluoxetine have an inter-mediate affinity for 5-HT_{2C} receptors (Palvimaki, E.P. *Psychopharmacology (Berl.)* **1996**, *126*, p 234-240). Moreover, it has been reported that chronic administration of citalopram, fluoxetine and paroxetine leads to functional desensitisation of 5-HT_{2C} receptors (Kennett, G.A. et al. *Neuropharmacology* **1994,** 33, p 1581-1588, Maj J. et al. *Psychopharmacology (Berl)* **1996,** *127,* p 73-82, and Quested, D.J. *Psychopharmacology (Berl.)* **1997**, *133*, 305-308). It has also been suggested that this change may contribute to the therapeutic efficacy in depression, anxiety disorders and OCD (obsessive compulsive disorder).

In a recent clinical study, it was shown that pindolol and mianserin augment the efficacy of fluoxetine in the treatment of treatment resistant patients and that mianserin may shorten the latency of onset of antidepressive effect when combined with fluoxetine. The effect of mianserin in this study can be explained by at least four different mechanisms relating to the effect of mianserin on noradrenaline turnover, α₂-receptor blockade and its antagonistic effect at 5-HT_{2A/2C} receptors, (Maes, M. et al. *J.Clin. Psychopharmacol,* **1999**, *19*, 2, p 177-182).

However, it has also been reported that in the mouse forced swimming test, prior administration of the selective 5-HT_{2A/2C} antagonists, ritanserin (4 mg/kg i.p.) or ketanserin (8 mg/kg i.p.) potentiated the effects of imipramine (mg/kg i.p.) and desipramine (16 mg/kg i.p.), but not of fluoxetine 16 mg/kg i.p.), citalopram (16 mg/kg i.p.) or fluvoxamine (8 mg/kg i.p.) (Redrobe, J.P. et al. *Eur J Pharmacol.* **1997,** *325*, 129-135).

It has now been found that the combination of a serotonin uptake inhibitor with a compound having 5-HT_{2C} antagonistic or inverse agonistic effect (compounds having a negative efficacy at the 5-HT_{2C} receptor) provides a considerable increase in the level of 5-HT in terminal areas, as measured in microdialysis experiments.

In particular, it has been found that ketanserin, ritanserin, RS 102221, ((+)-trans-1-(5-chloro-3-(4-fluorophenyl)-1-indanyl)-4-(2-(3-isopropyl-2-imidazolidinon-1-yl)ethyl)-piperazine and 2,5-dimethyl-3-(4-fluorophenyl)-1-[1-[2-(imidazolidin-2-on-1-yl)ethyl]-pipezidin-4-yl]-1H-indole in combination with SSRIs, *synergistically* act to increase the level of extracellular serotonin. As applied to humans, this would imply a shorter onset of antidepressant effect in the clinic and an augmentation, or potentiation of the therapeutic effect of the serotonin reuptake inhibitor (SRI).

### The invention

The present invention thus provides:

The use of a 5-HT_{2C} receptor antagonist, inverse agonist or partial agonist for the preparation of a pharmaceutical composition to be used in combination with a serotonin reuptake inhibitor inhibitor.

In particular, the present invention relates to the use of a 5-HT_{2C} receptor antagonist, inverse agonist or partial agonist for the preparation of a pharmaceutical composition useful for augmenting and/or providing faster onset of the therapeutic effect of a serotonin reuptake inhibitor.

In a preferred embodiment, the invention relates to the use as above wherein the serotonin reuptake inhibitor is used for the treatment of depression, anxiety disorders and other affective disorders, including generalized anxiety disorder, panic anxiety, obsessive compulsive disorder, acute stress disorder, post traumatic stress disorder or social anxiety disorder, eating disorders such as bulimia, anorexia and obesity, phobias, dysthymia, premenstrual syndrome, cognitive disorders, impulse control disorders, attention deficit hyperactivity disorder, drug abuse and any other disorder responsive to a SRI.

In another embodiment, the invention relates to the use of
b) a combination of a compound, which is a serotonin reuptake inhibitor, and a compound, which is a 5-HT_{2C} receptor antagonist, inverse agonist or partial agonist,
for the preparation of a pharmaceutical composition or kit useful for the treatment of depression, anxiety disorders and other affective disorders, such as generalized anxiety disorder, panic anxiety, obsessive compulsive disorder, acute stress disorder, post traumatic stress disorder and social anxiety disorder, eating disorders such as bulimia, anorexia and obesity, phobias, dysthymia, premenstrual syndrome, cognitive disorders, impulse control disorders, attention deficit hyperactivity disorder, drug abuse or any other disorder responsive to serotonin reuptake inhibitors.

In a further embodiment, the invention relates to a pharmaceutical composition or kit comprising:
b) a combination of a compound, which is a serotonin reuptake inhibitor, and another compound, which is a 5-HT_{2C} receptor antagonist, inverse agonist or partial agonist,
and optionally pharmaceutically acceptable carriers or diluents.

In a particular embodiment, a selective serotonin reuptake inhibitor is used according to the invention.

In another particular embodiment, a compound which is selective for the 5-HT_{2C} receptor is used according to the invention.

In a further embodiment, a compound which is an antagonist, an inverse agonist at the 5-HT_{2C} receptor is used according to the invention.

The pharmaceutical composition or kit according to the invention may be adapted for simultaneous administration of the active ingredients, or it may be adapted for sequential administration of the active ingredients.

When the pharmaceutical composition is adapted for simultaneous administration, the active ingredients may be contained in the same unit dosage form.

When the pharmaceutical composition or kit is adapted for sequential administration, the active ingredients are contained in discrete dosage forms, optionally contained in the same container or package.

In particular, the present invention relates to the use of, and to pharmaceutical compositions or kits comprising the following combinations:

Deramciclane and an SRI selected form citalopram, escitalopram, fluoxetine, sertraline, paroxetine, fluvoxamine, venlafaxine, dapoxetine, nefazodone, imipramin, femoxetine and clomipramine,

According to the invention, it has been found that co-administration of 5-HT_{2C} receptor antagonist or inverse agonist and a serotonin reuptake inhibitor produces a significant increase in the level of serotonin in terminal areas, as measured in microdialysis experiments, compared to the administration of the serotonin reuptake inhibitor alone.

Administration of RS 102221, (+)-trans-1-(5-chloro-3-(4-fluorophenyl)-1-indanyl)-4-(2-(3-isopropyl-2-imidazolidinon-1-yl)ethyl)-piperazine, 2,5-dimethyl-3-(4-fluorophenyl)-1-[1-[2-(imidazolidin-2-on-1-yl)ethyl]-piperidin-4-yl]-1H-indole and ketanserin alone causes no increase in serotonin levels as measured in microdialysis experiments.

According to the invention, animal studies have shown that 5-HT_{2c} receptor antagonist or inverse agonist may provide fast onset of therapeutic effect of serotonin reuptake inhibitors and potentiate the anxiolytic potential of serotonin reuptake inhibitors.

The use of a combination of the 5-HT_{2C} receptor antagonist, inverse agonist or partial agonist and a serotonin reuptake inhibitor may greatly reduce the amount of serotonin reuptake inhibitor necessary to treat depression and other affective disorders and may thus reduce the side effects caused by the serotonin reuptake inhibitor. In particular, the combination of a reduced amount of SRI and a 5-HT_{2C} receptor antagonist, inverse agonist or partial agonist may reduce the risk of SSRI-induced sexual dysfunction and sleep disturbances.

Co-administration the 5-HT_{2C} receptor antagonist, inverse agonist or partial agonist and a serotonin reuptake inhibitor may also be useful for the treatment of refractory depression, i.e. depression, which cannot be treated appropriately by administration of a serotonin reuptake inhibitor alone. Typically, 5-HT_{2c} receptor antagonist, inverse agonist or partial agonist may be used as add-on therapy for the augmentation of the response to SRIs in patients where at least 40-60% reduction in symptoms has not been achieved during the first 6 weeks of treatment with an SRI.

Many antidepressants with serotonin reuptake inhibiting effect have been described in the literature. Any pharmacologically active compound, which primarily or partly exert its therapeutic effect via inhibition of serotonin reuptake in the CNS, may benefit from augmentation with a 5-HT_{2C} receptor antagonist, inverse agonist or partial agonist.

The following list contains a number of serotonin reuptake inhibitors, which may benefit from augmentation with the 5-HT_{2C} receptor antagonist, inverse agonist or partial agonist: citalopram, escitalopram, fluoxetine, R-fluoxetine, sertraline, paroxetine, fluvoxamine, venlafaxine, duloxetine, dapoxetine, nefazodone, imipramine, imipramine N-oxide, desipramine, pirandamine, dazepinil, nefopam, befuraline, fezolamine, femoxetine, clomipramine, cianoimipramine, litoxetine, cericlamine, seproxetine, WY 27587, WY 27866, imeldine, ifoxetine, tiflucarbine, viqualine, milnacipran, bazinaprine, YM 922, S 33005, F 98214-TA, OPC 14523, alaproclate, cyanodothepine, trimipramine, quinupramine, dothiepin, amoxapine, nitroxazepine, McN 5652, McN 5707, VN 2222, L 792339, roxindole, YM 35992, Ol 77, Org 6582, Org 6997, Org 6906, amitriptyline, amitriptyline N-oxide, nortriptyline, CL 255.663, pirlindole, indatraline, LY 113.821, LY 214.281, CGP 6085 A, RU 25.591, napamezole, diclofensine, trazodone, EMD 68.843, BMY 42.569, NS 2389, sercloremine, nitroquipazine, ademethionine, sibutramine, clovoxamine. The compounds mentioned above may be used in the form of the base or a pharmaceutically acceptable acid addition salt thereof.

The above list of serotonin reuptake inhibitors and other compounds, which cause an increase in the extracellular level of serotonin, may not be construed as limiting.

SRIs, which are particularly preferred according to the present invention, include citalopram, escitalopram, fluoxetine, sertraline, paroxetine, fluvoxamine, venlafaxine, dapoxetine, nefazodone, imipramin, femoxetine and clomipramine.

The term selective serotonin reuptake inhibitor (SSRI) means an inhibitor of the monoamine transporters which has stronger inhibitory effect at the serotonin transporter than the dopamine and the noradrenaline transporters. Particularly preferred SSRIs according to the invention are citalopram, escitalopram, fluoxetine, fluvoxamine, sertraline and paroxetine.

In a preferred embodiment, a 5-HT_{2c} receptor ligand selected from deramciclane, used according to the invention.

The Ki values at the 5-HT_{2c} receptor for some of the compounds mentioned in the list above is given in the table below:

| **Compound** | **Ki (nM)** |
|---|---|
| ketanserine | 14 |
| irindalone | 19 |
| Lu 29066 | 6.9 |
| Lu 27121M | 5.1 |
| fluoxetine | 80 |
| nefazodone | 33 |
| RS 102221 | 0.56 |
| ritanserin | 0.19 |
| mianserin | 1.1 |

The Ki values given in the table above were determined using the 5-HT_{2C} binding assay described in the experimental section. It should be noted that other tests, modified tests or tests carried out by other groups may result in figures that differ somewhat from the figures given in the table above.

As used herein, the term selective for the 5-HT_{2C} receptor means that the compound possess higher affinity for the 5-HT_{2C} receptor than to other serotonin receptors.

Further 5-HT_{2C} receptor antagonists or inverse agonists are described e.g. in WO 97/39001, WO 97/37989, WO 97/08167, WO96/39382, WO 96/23769, WO 96/11930, WO 95/01976, WO 94/14801, WO 94/04533, *Bromidge et al., J. Med. Chem.,* **2000**, *43*, 1123-113 and Bøgesø et al. *Schizophrenia* **1995,** Alfred Benzon Symposium **38,** pp 361-374.

In a particular embodiment of the invention the compounds used according to the invention has a Ki value at the 5-HT_{2c} receptor, which is below 30 nM and an IC₅₀ value at the serotonin transporter, which is below 20 nM.

When a combined serotonin reuptake inhibitor and 5-HT_{2C} antagonist, inverse agonist or partial agonist is used according to the invention , the compound is preferably lacking affinity for the dopamine receptor, i.e it has an IC₅₀ value over 100 nM at this receptor.

The active ingredients according to the invention, may be used in the free base form or in the form of a pharmaceutically acceptable acid addition salt thereof, the latter being obtainable by reaction of the base form with an appropriate acid. Exemplary of organic acid addition salts according to the invention are those with maleic, fumaric, benzoic, ascorbic, succinic, oxalic, bis-methylenesalicylic, methanesulfonic, ethanedisulfonic, acetic, propionic, tartaric, salicylic, citric, gluconic, lactic, malic, mandelic, cinnamic, citraconic, aspartic, stearic, palmitic, itaconic, glycolic, p-aminobenzoic, glutamic, benzenesulfonic, and theophylline acetic acids, as well as the 8-halotheophyllines, for example 8-bromotheophylline. Exemplary of inorganic acid addition salts according to the invention are those with hydrochloric, hydrobromic, sulfuric, sulfamic, phosphoric, and nitric acids.

The term addition salt as used herein above also comprises the solvates, which the active ingredients are able to form. Said solvates are meant to be included within the scope of the present invention. Examples of such solvates are e.g. the hydrates, alcoholates and the like.

Citalopram is preferably used in the form of the hydrobromide or as the base, escitalopram in the form of the oxalate, fluoxetine, sertraline and paroxetine in the form of the hydrochloride and fluvoxamine in the form of the maleate.

The amount of 5-HT_{2c} antagonist, inverse agonist or partial agonist in combination therapy may range from about 0.1 to about 150 mg/day, particularly from about 0.1 to about 100 mg/day and more particularly from about 0.5 to about 50 mg/day and even more particularly from about 1 to about 5 mg/day.

Serotonin reuptake inhibitors, including the SSRIs specifically mentioned herein above, differ both in molecular weight and in activity. As a consequence, the amount of serotonin reuptake inhibitor used in combination therapy depends on the nature of said serotonin reuptake inhibitor. The normal dose range of commercially available SRIs is well known. In one embodiment of the invention, the serotonin reuptake inhibitor or the compound causing an increase in the level of extracellular 5-HT, is administered at lower doses than required when the compound is used alone. In another embodiment, the serotonin reuptake inhibitor or the compound causing an increase in the level of extracellular 5-HT, is administered in normal doses.

To prepare the pharmaceutical compositions of this invention, an effective amount of the active ingredients, in acid or base addition salt form or base form, is combined in intimate admixture with a pharmaceutically acceptable carrier, which can take a wide variety of forms depending on the form of preparation desired for administration. These pharmaceutical compositions are desirably in unitary dosage form suitable, for administration orally, nasal, rectally, percutaneously or by parenteral injection. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions; or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included.

It is especially advantageous to formulate the aforementioned pharmaceutical compositions in dosage unit form for ease of administration and uniformity of dosage. Dosage unit form as used in the specification and claims herein refer to physically discrete units suitable as unitary dosages, each unit containing a predetermined quantity of active ingredient calculated to produce the desired therapeutic effect, in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

The 5-HT_{2C} antagonist, partial agonist or inverse agonist may be administered before, during or after the administration of the serotonin reuptake inhibitor provided that the time between the administration of the 5-HT_{2C} antagonist, inverse agonist or partial agonist and the administration of the serotonin reuptake inhibitor is such that ingredients are allowed to act synergistically on the CNS.

When simultaneous administration of 5-HT_{2C} antagonist, partial agonist or inverse agonist and a serotonin reuptake inhibitor is envisaged, a composition containing both a serotonin reuptake inhibitor and 5-HT_{2C} antagonist, partial agonist or inverse agonist may be particularly convenient. The compositions may be prepared as described herein above.

Simultaneous administration may also be accomplished by administration of the active ingredients in two separate unit dosage forms.

When sequential administration of 5-HT_{2C} receptor antagonists, inverse agonists or partial agonists is envisaged, the pharmaceutical composition may comprise, for example, a kit including discrete unit dosage forms containing 5-HT_{2C} antagonist, partial agonist or inverse agonist and discrete unit dosage forms containing a serotonin reuptake inhibitor, all contained in the same container or pack, e.g. a blister pack.

### Experimental section

### 5-HT_{2C} binding

Ki values at the 5-HT_{2C} were determined by testing the compounds according to the procedure described below: SR-3T3 cells (ATCC CRL 9651) are grown to confluency and then scraped off in TBS (50 mM Tris, pH 7.7, 125 mM NaCl). The cell suspension is centrifuged and the pellet resuspended in 50 mM Tris, HCI, pH 7.7 and stored at -80°C. For experimental use, the frozen cell pellet is thawed and homogenised with an Ultra Turrax for 10-15 seconds. 1.5 mg cell preparation is incubated with 0.5 nM 3H-Mesulergine for 60 minutes at 37 °C. Incubation is terminated by rapid filtration through Filtermat B filters and bound radioactivity is determined in a Tricarb scintillation counter.

### 5-HTT inhibition

IC₅₀ values for serotonin reuptake inhibition are measured as described in the test below: Male rats (125-225 g) are decapitated and encephalon is dissected out and transferred to 0.9 % NaCl at 0 °C. The brain is weighed and homogenized at 0°C in a glass/teflon homogenizer in 40 volumes of 0.32 sucrose containing I nM Nialamid. The homogenate is centrifuged at 600 g for 10 minutes at 4°C. The supematant/S1 is centrifuged at 20000 g for 55 min at 4°C. Supernatant/S2 is discarded and the pellet is resuspended in 360 volumes of modified Krebs Ringer phosphate buffer, pH 7.4 (oxygenated for 10 minutes). The suspension is kept on ice. The tissue is incubated with ³H-5-HT for 15 minutes at 37 °C in Krebs Ringer buffer. The samples are filtered through 0.1 % PEI soaked Filtermat C filters to wash away ³H-5-HT in solution. Filters are washed with 10 µM 5-HT before and after sample filtration. Radioactivity captured in synaptosomes is counted in a Tricarb scintillation counter.

### PI Assay testing of the human 5-HT_{2c} receptor

Method : Cells are seeded in 96 well plate to give a density of 7 × 10⁴ cells per well (200 µl of medium) on day of experiment. Cells were loaded with 4 µCi myo-[2-³H]Inositol with PT6-271 stabilizer (Amersham Pharmcia Biotech) per well 24 hours before the experiment was run.

Column Preparation: Amiprep minicolumns with SAX Quartenary amine RPN.1908 is activated with 1 mL 1.0 M KHCO₃. Excess KHCO₃ is removed by washing by washing 1 mL of water through the column twice. 0.9 mL of water is added to each column ready for the addition of 200 µL of sample.

Cell Plate Procedure: Cells are washed three times with 200 µl KRH-buffer (KCI (5 mM), NaCl (124 mM), Glucose (8 mM) BSA (0,5 g/liter), MgSO4 , 7 H2O (1,25mM), HEPES (25mM), KH2PO4 (1,25mM), CaCl2 (1,45mM)). Cells are incubated in 200 µl KRH-LiCl-buffer (KRH-buffer 10 mM LiCl) for 30 minutes at 37°C.
1. For testing of agonists, replace buffer with agonists (200µl diluted in KRH -LiCl Buffer) and incubate at 37°C for 10 minutes. Proceed to step # 6.
2. For testing of inverse agonists, replace buffer with inverse agonists (200µl diluted in KRH -LiCl Buffer) and incubate at 37°C for 30 minutes. Proceed to step # 6.
3. For testing of antagonists, replace buffer with antagonists (200µl diluted in KRH - LiCl Buffer) and incubate at 37°C for 10 minutes. Then add agonist to each well except those used to define basal activity and incubate at 37°C for 10 minutes.
4. Aspirate off the solution from each well and add 200 µL of cold PCA (4 % HClO₄).
5. Incubate at 4°C for 15-30 minutes to allow inositolphosphate release from cells.
6. Add 46 µL of KOH-HEPES buffer (1.5 mM KOH - 60 mM HEPES buffer, pH~ 7.5) and incubate at 4°C for another 10 minutes.
7. Add 200 µL of the neutralised inositolphosphate solution from each well to the prepared columns and wash through.
8. Wash the columns with 1 mL of water.
9. Add 1 mL 0.05 M KHCO₃ and collect in scintillation tubes.
   - Add 4 mL of scintillation counting fluid (Ultima Gold™ scintillation counting fluid from Packard)
10. and count for 5 minutes on a Packard Tri-carb 2100TR Liquid Scintillation Analyzer (Program 4). Totals - Defined by 10nM 5-HT. Basal Activity - Defined by buffer.

The above three methods may be used in the method according to the invention for the identification of compounds for the treatment of diseases or disorders responsive to serotonin reuptake inhibitors. However, other methods and variants of these methods may also be used.

### Microdialysis Experiments

Male albino rats of a Wistar-derived strain (285-320 g; Harlan, Zeist, The Netherlands) were used for the experiments. The rats were housed in plastic cages (35 x 35 x 40 cm), and had free access to food and water. The experiments are concordant with the declarations of Helsinki and were approved by the Animal Care Committee of the Faculty of Mathematics and Natural Science of the University of Groningen.

The following drugs were used: Citalopram hydrobromide and internal standard (kindly donated by Lundbeck (Copenhagen, Denmark), courtesy Dr. Sanchez), ritanserin and ketanserin tartrate (RBI, Natick, USA). Citalopram and ketanserin were dissolved in saline, ritanserin was dissolved in 30% solutol/saline. Ketanserin and citalopram were injected subcutaneously (in different areas during co-administration), ritanserin was injected intraperitoneally. All drugs were administered in a volume of 1 mL per kg.

Microdialysis experiments were performed using home made I-shaped probes, made of polyacrylonitrile / sodium methyl sulphonate copolymer dialysis fiber (i.d. 220 µm, o.d. 0.31 µm, AN 69, Hospal, Italy). The exposed length of the membranes was 4 mm. Preceding surgery, rats were anaesthetised either by means of an intraperitoneal injection of 400 mg/kg chloral hydrate (ritanserin experiments) or by an intraperitoneal injection of ketamine/xylazin, with midazolam as premedication (ketanserin experiments). Lidocaine-HCl, 10% (m/v) was used for local anaesthesia. Rats were placed in a stereotaxic frame (Kopf, USA), and probes were inserted into the ventral hippocampus (coordinates: IA: +3.7 mm, lateral : +4.8 mm, ventral: -8.0 mm from the dura mater, Paxinos and Watson, 1982) and secured with dental cement.

In the pharmacokinetic experiments, blood was drawn through a canula made of silicon tubing, which was inserted into the right jugular vein for 3.8 cm. The tubing was transferred subcutaneously to the scull of the rat, and a stainless steel inlet was connected to the tubing. The inlet was mounted on the skull with dental cement and surgical screws. After insertion, canulas were filled with a PVP solution (55% polyvinylpyrrolidon in 500 IE/mL heparin) in saline to prevent blood clotting.

Pharmacokinetic experiments : Rats were allowed to recover for at least 24 h. Blood samples (0.30 mL) were drawn at 0,15,30, 60,120,240 and 360 min after injection of either citalopram or ketanserin. Samples were transferred to 1.5 mL eppendorf vials, containing 5 µL heparin (500 IE/mL saline), mixed and immediately transferred to a chilled centrifuge (MSE, England), and centrifuged for 15 min at 3,000 rpm.

Microdialysis experiments: Rats were allowed to recover for at least 24 h. Probes were perfused with artificial CSF containing 147 mM NaCl, 3.0 mM KCl, 1.2 mM CaCl₂, and 1.2 mM MgCl₂, at a flow-rate of 1.5 µL / min (Harvard apparatus, South Natick, Ma., USA). Samples were collected on-line in a 20 µL loop and injected automatically onto the column every 15 min.

Serotonin analysis: 5-HT was analysed using HPLC with electrochemical detection. The HPLC pump (Shimadzu LC-10 AD liquid chromatograph) was connected to a reversed phase column (phenomenex hypersil 3 : 3 µm, 100 x 2.0 mm, C18, Bester, Amstelveen, the Netherlands) followed by an electrochemical detector (Antec Leyden, Leiden, the Netherlands), working at a potential setting of 500 mV vs. Ag/AgCl reference. The mobile phase consisted of 5 g/L di-ammoniumsulfate, 500 mg/L ethylene diamino tetra acetic acid (EDTA), 50 mg/L heptane sulphonic acid and 30 µl/L of triethylamine, at a pH of 4.65 and 4.5% (w/w) methanol and water. Flow-rate of the mobile phase was 0.4 ml/min. The detection limit was 0.5 fmol 5-HT per 20 µL sample (signal to noise ratio 3).

Citalopram analysis: Citalopram was measured according to ⌀yehaug et al. (1982) Journal of Chromatography, 227; 129-135, with minor modifications. Briefly, to 150 µL plasma samples 75 µL of the internal standard LU 10-202 (2 µM) and 30 µL of 0.1 N NaOH were added. Samples were extracted twice by mechanically shaking for 3 minutes with 3 mL of diethyl ether. The ether layers were then transferred to 10 mL evaporating tubes, and 150 µL of 0.1 N HCI was added. The ether was evaporated in a water-bath at 40 °C under a stream of nitrogen. The HCI layer was washed once with 0.5 mL ether. 50 µL samples were injected onto the column.

With the experiments in which 5 mL plasma was obtained via the vacutainer, a similar protocol was used, but the plasma sample was now extracted three times with 10 mL of ether. An HPLC/auto-injector (1084B Liquid Chromatograph, Hewlett-Packard) was used, in combination with a fluorescence detector (470 Scanning Fluorescence detector, Waters, England) operating at an absorption wavelength of 240 nm, an emission wavelength of 296 nm, and a slit-width of 12 nm. Separation was performed using a Supelcosil HPLC column (5 µm, C 18, 250 x 46 mm, Supelco, the Netherlands), at ambient temperature. The mobile phase consisted of 46% v/v acetonitrile, 54% v/v potassium dihydrogen phosphate buffer (4.3 g/L), and 30 µl/L triethylamine, at a pH value of 3.0. Flow-rate in this system was 0.75 mL/min. The detection limit of the assay was 8 nM (signal to noise = 2). Extraction of samples spiked with known concentrations of citalopram revealed that the extraction method had a recovery of 99%.

Ketanserin analysis: Ketanserin was measured using liquid-liquid extraction, followed by reversed phase chromatography in conjunction with fluorescence detection. Briefly, to 150 µL plasma samples 30 µL of 0.1N NaOH were added. Samples were extracted twice by mechanically shaking for 3 minutes with 3 mL of diethyl ether. The ether layers were then transferred to 10 mL evaporating tubes, and 150 µL of 0.1 N HCl was added. The ether was evaporated in a water-bath at 40 °C under a stream of nitrogen. The HCl layer was washed once with 0.5 mL ether. 50 µL samples were injected onto the column. An HPLC/auto-injector (1084B Liquid Chromatograph, Hewlett-Packard) was used, in combination with a fluorescence detector (470 Scanning Fluorescence detector, Waters, England) operating at an absorption wavelength of 240 nm, an emission wave length of 370 nm, and a slit-width of 12 nm. Separation was performed using a Supelcosil HPLC column (5 µm, C18, 250 x 46 mm, Supelco, the Netherlands), at ambient temperature. The mobile phase consisted of 35% v/v acetonitrile, 65% v/v potassium dihydrogen phosphate buffer (4.3 g/L), and 30 µL/L triethylamine, at a pH value of 3.0. Flow-rate in this system was 1.0 mL/min. The detection limit of the assay was 6 nM (signal to noise = 2). Extraction of samples spiked with known concentrations of citalopram revealed that the extraction method had a recovery of 93%.

Data presentation and statistics: Four consecutive microdialysis samples with less then 20% variation were taken as control and set at 100%. Data are presented as percentages of control level (mean ± SEM). Statistical analysis was performed using Sigmastat for windows (Jandel Corporation). Treatment effects were compared using two way ANOVA for repeated measurements, followed by Dunnett's test. Level of significance level was set at *p*<0.05. Pharmacokinetic data were fitted using Multifit (copyright Dr. J.H. Proost, Dept. of pharmacokinetics and drug delivery, University of Groningen, the Netherlands).

### Results:

### Pharmacokinetic experiments:

### Citalopram administration

Upon subcutaneous administration of 10 µmol/kg citalopram initial plasma levels of around 0.7 µM developed, after which levels declined with an apparent half life of around 2 hours.

The following combinations of compounds were also tested as described above: Citalopram and RS 102221, citalopram and (+)-trans-1-(5-chloro-3-(4-fluorophenyl)-1-indanyl)-4-(2-(3-isopropyl-2-imidazolidinon-1-yl)ethyl)-piperazine, citalopram and 2,5-dimethyl-3-(4-fluorophenyl)-1-[1-[2-(imidazolidin-2-on-1-yl)ethyl]-piperidin-4-yl]-1H-indole and fluoxetin and ketanserin.

## Claims

1. The use of a combination of a compound, which is a serotonin reuptake inhibitor, and deramciclane, for the preparation of a pharmaceutical composition useful for the treatment of depression, anxiety disorders and other affective disorders, such as generalized anxiety disorder, panic anxiety, obsessive compulsive disorder, acute stress disorder, post traumatic stress disorder and social anxiety disorder, eating disorders such as bulimia, anorexia and obesity, phobias, dysthymia, premenstrual syndrome, cognitive disorders, impulse control disorders, attention deficit hyperactivity disorder, drug abuse or any other disorder responsive to serotonin reuptake inhibitors.

2. The use according to claim 1, where a selective serotonin reuptake inhibitor is used.

3. The use according to claims 1- 2 wherein the SRI is elected from citalopram, escitalopram, fluoxetine, sertraline, paroxetine, fluvoxamine, venlafaxine, dapoxetine, nefazodone, imipramin, femoxetine and clomipramine.

4. A pharmaceutical composition comprising a combination of a compound which is a serotonin reuptake inhibitor, and deramciclane and optionally pharmaceutically acceptable carriers or diluents.

5. A pharmaceutical composition according to claim 4 wherein the serotonin reuptake inhibitor used is a selective serotonin reuptake inhibitor.

6. A pharmaceutical composition according to claims 4 and 5 **characterized in that** the serotonin uptake inhibitor is selected from citalopram, escitalopram, fluoxetine, sertraline, paroxetine, fluvoxamine, venlafaxine, dapoxetine, nefazodone, imipramin, femoxetine and clomipramine.

7. A pharmaceutical composition according to claim 4-6 wherein the pharmaceutical composition is adapted for simultaneous administration of the active ingredients.

8. The pharmaceutical composition according to claim 4-7 wherein the active ingredients are contained in the same unit dosage form.

9. A pharmaceutical composition according to claim 4-6 wherein the pharmaceutical composition is adapted for sequential administration of the active ingredients.

10. The pharmaceutical composition according to claims 4-6 and 9 wherein the active ingredients are contained in discrete dosage forms.

## Patentansprüche

1. Verwendung einer Kombination aus einer Verbindung, die ein Serotoninwiederaufnahmeinhibitor ist, und Deramciclan zur Herstellung einer pharmazeutischen Zusammensetzung, die nützlich zur Behandlung von Depression, Angststörungen und anderen affektiven Störungen wie generalisierte Angststörung, panischer Angstzustand, obsessive Zwangsstörung, akute Belastungsstörung, posttraumatische Belastungsstörung und soziale Angststörung, Eßstörungen wie Bulimie, Anorexie und Fettsucht, Phobien, Dysthymie, prämenstruelles Syndrom, kognitive Störungen, Impulskontrollstörungen, Aufmerksamkeitsdefizit-Hyperaktivitätsstörung, Drogenmißbrauch oder jeder anderen Störung ist, die auf Serotoninwiederaufnahmeinhibitoren anspricht.

2. Verwendung gemäß Anspruch 1, worin ein selektiver Serotoninwiederaufnahmeinhibitor verwendet wird.

3. Verwendung gemäß Ansprüchen 1-2, worin der SRI aus Citalopram, Escitalopram, Fluoxetin, Sertralin, Paroxetin, Fluvoxamin, Venlafaxin, Dapoxetin, Nefazodon, Imipramin, Femoxetin und Clomipramin ausgewählt wird.

4. Pharmazeutische Zusammensetzung, die eine Kombination aus einer Verbindung, die ein Serotoninwiederaufnahmeinhibitor ist, und Deramciclan und gegebenenfalls pharmazeutisch akzeptablen Trägern oder Verdünnungsmitteln umfaßt.

5. Pharmazeutische Zusammensetzung gemäß Anspruch 4, worin der verwendete Serotoninwiederaufnahmeinhibitor ein selektiver Serotoninwiederaufnahmeinhibitor ist.

6. Pharmazeutische Zusammensetzung gemäß Ansprüchen 4 und 5, **dadurch gekennzeichnet, daß** der Serotoninwiederaufnahmeinhibitor aus Citalopram, Escitalopram, Fluoxetin, Sertralin, Paroxetin, Fluvoxamin, Venlafaxin, Dapoxetin, Nefazodon, Imipramin, Femoxetin und Clomipramin ausgewählt ist.

7. Pharmazeutische Zusammensetzung gemäß Anspruch 4-6, worin die pharmazeutische Zusammensetzung zur gleichzeitigen Verabreichung der aktiven Bestandteile angepaßt ist.

8. Pharmazeutische Zusammensetzung gemäß Anspruch 4-7, worin die aktiven Bestandteile in der gleichen Einheitsarzneiform enthalten sind.

9. Pharmazeutische Zusammensetzung gemäß Anspruch 4-6, worin die pharmazeutische Zusammensetzung zur aufeinanderfolgenden Verabreichung der aktiven Bestandteile angepaßt ist.

10. Pharmazeutische Zusammensetzung gemäß Ansprüchen 4-6 und 9, worin die aktiven Bestandteile in diskreten Arzneiformen enthalten sind.

## Revendications

1. Utilisation d'une combinaison d'un composé qui est un inhibiteur de recaptage de la sérotonine, et de déramciclane, en vue de la préparation d'une composition pharmaceutique utile dans le traitement de la dépression, des troubles de l'anxiété et autres troubles affectifs, tels que les troubles généralisés dus à l'anxiété, la peur panique, les troubles compulsifs obsessionnels, troubles de stress aigus, troubles de stress post-traumatiques et troubles d'angoisse en société, troubles nutritionnels tels que la boulimie, l'anorexie et l'obésité, les phobies, la dysthymie, le syndrome prémenstruel, les problèmes cognitifs, les troubles de la maîtrise des impulsions, les troubles d'hyperactivité avec déficit de l'attention, les abus de drogues et tout autre trouble susceptible de répondre aux inhibiteurs de recaptage de la sérotonine.

2. Utilisation selon la revendication 1, dans laquelle on utilise un inhibiteur de recaptage de sérotonine sélectif.

3. Utilisation selon les revendications 1-2, dans laquelle l'inhibiteur de recaptage de sérotonine est choisi à partir de citalopram, escitalopram, fluoxétine, sertraline, paroxétine, fluvoxamine, venlafaxine, dapoxétine, néfazodone, imipramine, fémoxétine et clomipramine.

4. Composition pharmaceutique comprenant une combinaison d'un composé qui est un inhibiteur de recaptage de la sérotonine, et de déramciclane et, facultativement, de supports ou diluants pharmaceutiquement acceptables.

5. Composition pharmaceutique selon la revendication 4, dans laquelle l'inhibiteur de recaptage de sérotonine utilisé est un inhibiteur de recaptage de sérotonine sélectif.

6. Composition pharmaceutique selon les revendications 4 et 5, **caractérisée en ce que** l'inhibiteur de recaptage de sérotonine est choisi à partir de citalopram, escitalopram, fluoxétine, sertraline, paroxétine, fluvoxamine, venlafaxine, dapoxétine, néfazodone, imipramine, fémoxétine et clomipramine.

7. Composition pharmaceutique selon la revendication 4-6, dans laquelle la composition pharmaceutique est apte à l'administration simultanée des ingrédients actifs.

8. Composition pharmaceutique selon la revendication 4-7, dans laquelle les ingrédients actifs sont contenus dans la même forme posologique unitaire.

9. Composition pharmaceutique selon la revendication 4-6, dans laquelle la composition pharmaceutique est apte à l'administration séquentielle des ingrédients actifs.

10. Composition pharmaceutique selon les revendications 4-6 et 9, dans laquelle les ingrédients actifs sont contenus dans des formes posologiques individuelles.
